**Europäisches Patentamt**

**European Patent Office**

**Office européen des brevets**

(11) Publication number: **0 105 937**
**B1**

(12) **EUROPEAN PATENT SPECIFICATION**

(45) Date of publication of patent specification: **19.11.87**

(21) Application number: **82109165.9**

(22) Date of filing: **04.10.82**

(51) Int. Cl.⁴: **C 12 P 7/10** // D21C3/06, C13K1/02

(54) Method of manufacturing alcohol from ligno-cellulose material.

(43) Date of publication of application:
**25.04.84 Bulletin 84/17**

(45) Publication of the grant of the patent:
**19.11.87 Bulletin 87/47**

(84) Designated Contracting States:
**DE GB**

(56) References cited:
**EP-A-0 044 658**
**WO-A-81/01154**
**FR-A- 465 534**
**GB-A- 120 520**
**GB-A-1 604 948**
**US-A-2 801 206**

**CHEMICAL ABSTRACTS, vol. 94, no. 10, May 1982, page 500, no. 154945Q, Columbus, Ohio, USA. R. E. BROOKS et al.: "Bioconversion of plant biomass to ethanol"**

**CHEMICAL ABSTRACTS, vol. 94, no. 6, February 1981, page 178, no. 33530s, Columbus, Ohio, USA. C. R. WILKE et al.: "Process development studies on the bioconversion of cellulose and production of ethanol"**

(73) Proprietor: **Strouth, Baron Howard Steven**
**El Madronal San Pedro de Alcantara**
**Malaga (ES)**

(72) Inventor: **Jelks, J. W.**
**4001 N. Highway 97**
**Sand Springs Oklahoma 74063 (US)**
Inventor: **Strouth, Baron Howard Steven**
**El Madronal San Pedro de Alcantara**
**Malaga (ES)**

(74) Representative: **Riederer Freiherr von Paar zu Schönau, Anton**
**Van der Werth, Lederer & Riederer Freyung 615**
**Postfach 2664**
**D-8300 Landshut (DE)**

(56) References cited:
**CHEMICAL ENGINEERING, vol. 88, no. 2, 26th January 1981, pages 51,53,55, McGraw Hill Public., New York, USA. "Wood-to-ethanol methods edge closer to fruition"**

## Description

This invention relates to a method of manufacturing alcohol and particularly relates to a method of producing alcohol utilizing cellulose bearing raw material containing lignin which is not normally employed for such purpose. Particularly, the field of the invention is directed towards the process of manufacturing alcohol utilizing, as a raw material, ligno-cellulose biomass derived from wood, straw, bagasse, seed hulls, nut hulls, etc., and an object of the invention is that such method may be a self-contained method for which a supplementary energy source for supplying heat is not required or greatly minimized.

Alcohol as used herein means ethyl alcohol, $C_2H_5OH$, also known as ethanol or grain alcohol. Ethanol has many uses. As the least toxic of all alcohols, it is used in alcoholic beverages. However, in recent years, due to the shortage of petroleum sources of energy, a renewed interest has been generated in the manufacture of ethanol as a fuel source for internal combustion engines. While ethanol may be produced synthetically from ethylene and acetylene, the raw materials for such synthetic production are hydrocarbons, and therefore producing ethanol from hydrocarbons does not contribute to the availability of liquid energy useable in internal combustion engines. Therefore, there has been renewed interest in the manufacture of ethanol by fermentation of plant products. The known techniques for producing ethanol by fermentation require the use of raw materials such as grains (corn being a most commonly employed raw material) which are readily digestible foods. Thus the present techniques of manufacturing ethanol by fermentation of organic material employ the use of products which deplete available food supply. Since there is in the world today, in addition to an energy shortage, a food shortage, the manufacture of alcohol using food products is merely a trade-off of one urgently needed material for another.

While there is a lack of sufficient organic material which is digestible by humans or animals in the world today, there is a large substantially untapped supply of organic material which is not digestible by humans or animals and which is not fermentable for the production of alcohol.

From CHEMICAL ABSTRACTS, Vol. 94, No. 10, page 500, No. 154945Q, Brooks, wood biodelignification and subsequent fermentation is known. $SO_2$/steam-treated poplar is used for fermentation. In CHEMICAL ABSTRACTS, Vol. 94, No. 6, page 178, No. 33530s, WILKE, conversion of wood by $SO_2$ is objected for reasons of poor effectivity; but studies of producing sugar from straw are mentioned. GB—A 120520 mentions the process of producing fermentable sugars from ligno-cellulose by the action of $SO_2$, without giving further details. WO 81/01154 (page 16) and US—A 2801206 (column 1) mention the possibility of burning lignin to provide steam for a process in which the lignin is a by-product.

The purpose of the present invention is to provide a means of efficiently and economically producing alcohol by fermentation utilizing as a raw material ligno-cellulose compositions which are not readily utilizable either for food or fermentation. Such materials include sawdust or other types of particulate wood matter derived from the trunk, bark, limbs, and leaves of trees and plants. Straw, bagasse, seed hulls, nut shells, etc. may also be efficiently used.

The distinction between digestible and non-digestible cellulose containing organic matter is primarily related to the lignin content. In order to provide strength in the cellular walls of plants, nature has arranged ligno-cellulose cellular structures. The lignin is somewhat characteristic of a glue or bond which holds cellulose material together to attain rigidity and stiffness. It is the lignin content of trees which makes them useable for structural purposes in the form of lumber. The lignin content of seed hulls and nut hulls provides a protective layer for the nutritious meat produced by the plants. The lignin content of straw enables wheat, rye, rice, etc. to support the grain product uprightly and to support the leafy portions of the plants for exposure to sunlight. Thus, most of the organic material produced by plants contains a high percentage of lignin and only a small percent is sufficiently free of lignin as to be digestible by humans or by animals.

The purpose of this invention is to provide a means of treating organic material having a high degree of lignin content in such a way as to separate the ligning material, leaving a cellulose product which is readily fermentable to produce alcohol.

The method of manufacturing ethanol, commonly referred to simply as alcohol, from ligno-cellulose material is characterized in claim 1. The woody raw material is first ground or chopped into particulate matter, preferably having a size of not more than 7 mm (1/4"); however, the finer the particulate matter is ground the more expediently the subsequent reactions take place. The particulate ligno-cellulose material is mixed with water and, under heat and pressure, is mixed with gaseous sulfur dioxide. The sulfur dioxide in the presence of water reacts with the lignin content of the ligno-cellulose material. The mixture is subsequently mixed with an alkaline solution which dissolves the reacted lignin material. The mixture is then washed to remove the dissolved lignin content and separate out the cellulose content of the mixture. This separated cellulose content is hydrolyzed and then fermented by the usual method. The product of the fermentation is alcohol (ethanol). By dehydration, the purity of the alcohol can be upgraded to that sufficient for use as a fuel in internal combustion engines. The removed lignin which is a waste product is dried to some degree and is then burned to provide for the energy of the process. The reaction of the particulate ligno-cellulose material, water, and sulfur dioxide, under heat and pressure, is preferably carried out in a rotary digester.

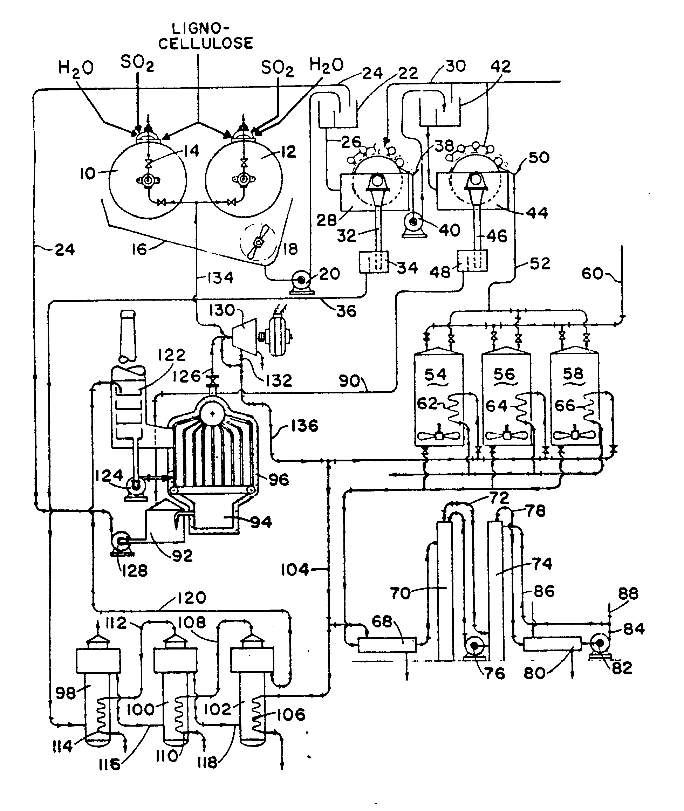

The drawing is a schematic arrangement of the process steps and apparatus which may be employed in practising the invention.

In the drawing, first and second globe rotary digesters 10 and 12 are shown. While only two digesters are shown, it is possible to use only a single digester or any number of digesters for the purposes of this invention. The use of more than one digester, however, is preferred since the reaction, which takes place within each of the digesters is a batch process. By using a plurality of digesters the balance of the procedure for manufacturing alcohol can be carried out as a continuous process. Each of the globe rotary digesters 10, 12 is an apparatus which is used to subject the contents thereof to heat and pressure while the contents is being mixed. Any device which accomplishes these results would be within the scope of the invention, whether carried out on a batch process or a continuous process.

Particulate ligno-cellulose material is deposited in digester 10 to substantially fill the digester, however leaving sufficient room so that the particulate material will mix as the digester rotates. The particulate ligno-cellulose may be any type of wood, straw, bagasse, nut hulls, seed hulls, etc. The woody material is first chopped or ground into particles. The size of the particles should be not more than approximately 7 mm (1/4'') but may be as fine as desired. The size of the particles is not important except that it is apparent that the smaller the particles the more expeditious will be the subsequent reaction. Thus the particle size is a matter of engineering trade-off between the cost of reducing the ligno-cellulose material to smaller sizes versus the time utilized in the steps of reaction.

After the ligno-cellulose material (hereinafter called wood) is deposited in digester 10, water and sulfur dioxide are added. The water is added to a ratio of approximately 40% by weight of water to the dry weight of the wood. Sulfur dioxide is added in the form of a gas at a ratio by weight of about 3 to 6% of the dry weight of the wood. The quantity of sulfur dioxide is predicated upon the percentage of lignin compared to cellulose contained in the wood. When the quantity of lignin is less, such as when cottonwood species is used as a raw material, compared to greater as when oak or pine are used as raw material, the quantity of sulfur dioxide is less. After the wood, sulfur dioxide and water have been added, the contents of the digester 10 are pressurized by the introduction of steam. Steam is introduced sufficient to bring the temperature of the reaction mixture to between 44°C and 82°C, a range between 49°C and 82°C being preferred, and at a pressure of between 9500 and 10500 hPa (135 and 150 psi). The mixture is cooked at this pressure and temperature for a period of about 40 minutes to 1 hour. However, the duration is determined by the particulate size of the wood raw material and the characteristics of the material.

. After a cooking cycle is completed in digester 10, a valve 14 is opened to allow steam to escape to the atmosphere. After the digester has been depressurized, the loading door is opened and the contents is discharged onto a digester pump pit 16. After the digester is emptied, it is refilled with wood particulate material, water, and sulfur dioxide and the process repeated. By use of a second digester 12 it can be seen that one digester may be in the process of being emptied and refilled while the other digester is in the cooking cycle so that reacted raw material is deposited into the digester pump pit 16 on a more frequent basis. By the use of a greater number of digesters the deposit can be even more frequent so that the digester pump pit 16 always contains a quantity of reacted material for subsequent processing on a continuous basis.

The reacted material in digester pit 16 is in the form of a slurry and, if necessary, additional water may be added to the pit to maintain the slurry as a pumpable mixture. The digester pump pit includes stirring apparatus 18 to make sure the reacted material does settle out and is retained as a pumpable slurry.

By means of a pump 20 the slurry is conducted to a mixing box 22 where the reacted mixture is mixed with an alkaline solution from line 24. The source of the alkaline solution which is conducted through line 24 will be described subsequently.

The reaction of the wood in digesters 10 and 12 is such that the sulphur dioxide in the presence of water reacts with the lignin content of the wood to enhance the solubility of the lignin content without destroying the cellulose content. Lignin is a fairly complex organic material, varying somewhat from one plant species to another and the exact reaction with sulfur dioxide is not precisely known; however, it has been experimentally verified that the reaction of the sulfur dioxide and wood produces a solution such that when mixed with an alkaline solution the ligning is dissolved.

The mixed reaction mixture and alkaline solution flow out of mixing box 22 through line 26 into a vacuum washer 28. This apparatus is a known type of equipment utilized in the chemical processing industry, and particularly in the paper making industry. Therefore, it is well understood by practitioners in the art and will not be described in detail. Suffice it to say that in a vacuum washer a cylinder is employed which is rotated in the solution. The interior of the cylinder is impressed with a vacuum. Water is sprayed onto the cylinder as it is above the solution to wash away the dissolved matter, the cellulose material being fibrous in nature, forms a mat on the cylinder. The fresh water utilized to wash the lignin content from the matted cellulose is supplied by line 30. The vacuum required by the washer 28 may be supplied by a vacuum pump or by means of, as illustrated, a barometric leg 32 which communicates with an overflow box 34. The dissolved lignin is carried out of the overflow box by line 36 while the cellulose is rolled off the vacuum forming cylinder into a receiving box 38. This matted material is still in a slurry form and is carried by a pump 40 into a second mixing box 42 where the cellulosic material is mixed with water from line 30. The washing procedure is repeated in a second vacuum washer 44 having a barometric leg 46 and overflow box 48 by which the overflow liquid content is carried away from the vacuum washer. The matted cellulose content is rolled off the cylinder into a receiving box 50. The content of receiving box 50 is a properly washed material which has more than 95% of the original lignin content removed.

While the use of vacuum washers 28 and 44 have been illustrated herein, it is understood that alternate washing procedures can be used in practice of the invention and that the particular means of washing the reacted ligno-cellulose material mixed with an alkaline solution is not a critical element in the practice of the invention and that other known washing systems may be employed. The essence of the washing step is simply to separate the dissolved lignin content from the cellulose material.

The cellulose content is conveyed by a line 52 to reactor-fermentors 54, 56, and 58. While three such reactor-fermenters are illustrated, it is apparent that more or less may be employed. The reactor-fermenters are a batch type operation. The reactor-fermenters are employed for enzymatic hydrolysis of the cellulose material, however, the invention is not limited to such type of hydrolysis. Acid hydrolysis may be used and in which case the equipment and the flow arrangement is somewhat altered. In the enzymatic hydrolysis as illustrated, a celluase enzyme is added by line 60. The pH of the solution within the reactor-fermenters is adjusted to approximately 4.5 plus or minus 0.3 and the temperature is corrected to be about 60°C (140°F). This is accomplished by use of steam heat exchangers 62, 64 and 66 in the reactor-digesters.

The material within the digesters is stirred constantly for a time sufficient to allow the complete enzymatic reaction, and such usually requires 12 to 24 hours, depending upon the type of cellulose derived from the raw material. After each of the reactors 54, 56, and 58 is completed, the reactor is cooled and cooling equipment, though not illustrated, may be employed. The mixture is preferably cooled to about 30°C (90°F). At which time yeast is added and the batch is allowed to ferment until the fermentable sugars are converted to alcohol and carbon dioxide. This reaction requires approximately three days more or less.

The number of reactor-fermenters is illustrated as three, but in the preferred practice of the process there is a sufficient number such that a continuous stream of beer can be furnished for distillation. The alcohol produced by fermentation within the reactor-fermenters has a high water content and, in fact, the alcohol content may be only about 15%. The beer is first passed through a heater 68 and then into a stripper or beer column 70. Additional heat is usually provided to the contents of column 70 such as by direct steam injection. Vapors from the distillation column 70, which will normally be about 50% alcohol and 50% water, pass by line 72 and pump into a rectifier column 74. By means of a pump 76, reflux from the rectifier volumn 74 is carried back into the beer column 70. Alcohol vapors from the rectifier column pass out through line 78 into a condenser 80. The output of the condenser is passed by means of pump 82 to an outlet 84 where it is split — part of the outlet stream being passed by line 86 back into the rectifier column of reflux purposes while the balance passes out through alcohol product line 88.

The method described to this point is a means of producing alcohol utilizing ligno-cellulose as a raw material. The additional elements needed in the process include sulfur dioxide, an alkaline solution, water, heat and steam. An important part of the invention is a means wherein the heat, steam and alkaline solution, are produced by the process of the invention.

The lignin solution from overflow box 48 is taken by line 90 to a dissolving tank 92. Melt from the melt pit 94 of a boiler 96 also passes into the dissolving tank 92.

The lignin solution from overflow box 34 is carried by line 36 to a set of multi-effective evaporators 98, 100, and 102. The number of such evaporators may be varied. The purpose of the evaporators is to evaporate the water content from the dissolved liquid solution. To obtain the heat necessary for the evaporation step, steam is applied from line 104 to a heat exchanger 106 in evaporator 102. Steam from evaporator 102 is passed by line 108 to a heat exchanger 110 in evaporator 100. In like manner, steam from evaporator 100 is passed by line 112 to a heat exchanger 114 in evaporator 98. The condensate from evaporator 98 is passed by line 116 into evaporator 100. The condensate from evaporator 100 is passed by line 118 into evaporator 98. The condensate from evaporator 102 is passed by line 120 into a contact evaporator 122. The lignin content of the solution from the overflow box 34 will normally be about 20% solids, more or less, and the balance liquid. This solution will be concentrated so that the ligning content passing from the multi-effective evaporators in line 120 will be about 55% more or less. In the contact evaporator 122 this solution is brought in contact with recovery boiler stack gases from boiler 96. The solution in the contact evaporator 122 is concentrated to about 65% solids which are carried into a burner 124. The organic solids burns in burner 124 and boiler 96 to produce significant quantities of heat. This heat is used to convert water in the boiler to steam which passes out through steam line 126. The inorganic portions of the burned material is primarily sodium and ends up as molten sodium carbonate in the melt pit 94 under boiler 96.

The molten sodium carbonate passes into the melt pit 94 and from thence into dissolving tank 92. When mixed with water in the dissolving tank an alkaline solution forms which is conveyed by pump 128 and line 24 into the mixing box 22. Thus the burned lignin content of the wood raw material is used to produce the alkaline solution for the process. Obviously, if additional alkaline solution is required, it may be supplied by supplementary means.

Steam from boiler 96 passing out through steam line 126 is conveyed to a turbine generator 130 to produce electrical energy. The turbine is preferably of the bleeder type which generates electricity and provides process steam by bleeding it from an intermediate point in the turbine at line 132. This process steam is carried by line 134 to the globe rotary digesters 10 and 12 and by line 136 to the heat exchangers 62, 64, and 66 of the reactor-fermentors. Line 136 is also connected with line 104 to provide heat to the multi-effect evaporator 102 and to the heater 68. Spent steam from the reactor-fermenters, the multi-effect evaporators, heaters 68, and turbine 130 may be collected by apparatus not shown and condensed to provide the water which is reused for input into the globe digesters, the mixing box 42, and for spray water on the washers 28 and 44.

The invention described provides the basic steps of the method and the basic components for practicing the invention. The invention provides a substantially self-contained method for manufacturing alcohol for use as a fuel out of non-digestible organic ligno-cellulose material in a manner wherein a supplementary energy source for supplying heat is not required or if supplemental energy is required it is greatly minimized. The method provides a means of increasing the availability of alcohol for use in internal combustion engines without utilizing digestible food material.

While the invention, as above indicated, has been described in certain detail, it is understood that the invention is not limited to the embodiments set forth herein but is to be limited by the scope of the attached claim or claims, including the full range of equivalency to which each element thereof is entitled.

Technique of conversion wood to saccharines is known per se, but the process of the invention yields, for example, 302 litres (80 gallons) of ethanol and 1 t of dry sawdust, out of 60 to 80% of the available material, the balance usable as animal feed, and uses the separated lignin as major energy source for processing.

## Claims

1. A self-contained method for manufacturing alcohol from particulate ligno-cellulose material such as wood, straw, bagasse, seed hulls, nut hulls, and the like, wherein a supplementary energy source for supplying heat is not required or is greatly minimized, to be carried out by the combination of following steps:

a) reacting the particulate ligno-cellulose material with sulfur dioxide and water under heat and pressure by introducing steam (in 10, 12);

b) mixing the reacted material from step a) with an alkaline solution to dissolve the reacted lignin (in 22);

c) washing the material from step b) to remove the dissolved lignin from the cellulose content of the mixture (in 28);

d) evaporating sufficient water from the dissolved lignin to provide a combustible fuel (in 98, 100, 102) and burning the combustible lignin in a boiler (96) to produce steam, which is recycled into step a) (via 134), and to produce sodium carbonate which is dissolved in water (in 92) and is then recycled into step b);

e) hydrolyzing the cellulose content from step c) (in 54, 56, 58); and

f) fermenting the hydrolyzed cellulose from step e) to produce alcohol (in 70, 74).

2. The method of claim 1, wherein in step a) the reaction mixture is composed of about 60% ligno-cellulose particulate by dry weight, 40% of water, and 2.16 to 4.32% sulfur dioxide.

3. The method of claim 2, wherein step a) is carried out at a temperature of between 49 and 82°C, at a pressure of between 9500 and 10500 hPa, and for a duration of from about 40 minutes to one hour.

4. The method of any of claims 1 to 3, wherein step c) is accomplished by means of a vacuum washer (28).

5. The method of any of claims 1 to 4, wherein step a) is carried out in a rotary digester (10, 12).

6. The method of any of claims 1 to 5, wherein step d) is carried out in a set of evaporators (98, 100, 102) arranged in series and containing heat exchangers heated by steam, the steam heating the first evaporator (102) being derived from the boiler (96) and the steam heating the following evaporators being derived from the preceding ones, the dissolved lignin being conveyed in the opposite direction.

7. The method of claim 6, wherein the dissolved lignin leaving the set of evaporators (98, 100, 102) is further dried in the stack gases of the boiler (96), before being burnt.

8. The method of any of claims 1 to 7, wherein the steam leaving the burner (96) is fed into a bleeder type turbine (130) for generating electricity and providing process steam.

## Patentansprüche

1. In sich geschlossenes Verfahren zum Herstellen von Alkohol aus kleinstückigem Lignozellulosematerial wir Holz, Stroh, Bagasse, Spreu, Nußschalen und dergleichen, bei dem eine zusätzliche Energiequelle zum Liefern von Wärme nicht oder nur in erheblich verkleinertem Umfang erforderlich ist, wobei das Verfahren durch die Kombination der folgenden Verfahrensschritte durchzuführen ist:

a) Bewirkung einer Reaktion des kleinstückigen Lignozellulosematerials mit Schwefeldioxid und Wasser unter Hitze und Druck durch Einleiten von Dampf (in 10, 12);

b) Mischen des der Reaktion unterworfenen Materials von Schritt a) mit einer alkalischen Lösung zum Auflösen des der Reaktion unterworfenen Lignins (in 22);

c) Waschen des Materials von Schritt b) zum Entfernen des gelösten Lignins vom Zellulosegehalt des Gemischs (in 28);

d) Verdampfen einer ausreichenden Wassermenge vom gelösten Lignin, um einen brennbaren Brenstoff zu ergeben (in 98, 100, 102), und Verbrennen des brennbaren Lignins in einem Heizkessel (92) zum Erzeugen von Dampf, der in Schritt a) rezirkuliert wird (über 134), und zum Erzeugen von Natriumkarbonat, das in Wasser gelöst wird (in 92) und dann in Schritt b) rezirkuliert wird;

e) Hydrolysieren des Zellulosegehalts von Schritt c) (in 54, 56, 58); und

f) Fermentieren der hydrolysierten Zellulose von Schritt e) zum Erzeugen von Alkohol (in 70, 74).

2. Verfahren nach Anspruch 1, wobei in Schritt a) das Reaktionsgemisch aus etwa 60 % kleinstückigem Lignozellulosematerial nach Trockengewicht, 40 % Wasser und 2,16 bis 4,32 % Schwefeldioxid zusammengesetzt ist.

3. Verfahren nach Anspruch 2, wobei Schritt a) bei einer Temperatur zwischen 49 und 82°C bei einem Druck zwischen 9500 und 10500 hPa für eine Dauer von etwa 40 Minuten bis einer Stunde durchgeführt wird.

4. Verfahren nach einem der Ansprüche 1 bis 3, wobei Schritt c) mit Hilfe eines Saugwäschers (28) durchgeführt wird.

5. Verfahren nach einem der Ansprüche 1 bis 4, wobei Schritt a) in einem rotierenden Zellstoffkocher (10, 12) durchgeführt wird.

6. Verfahren nach einem der Ansprüche 1 bis 5, wobei Schritt d) in einer Gruppe von Verdampfern (98, 100, 102) durchgeführt wird, die in Reihe angeordnet sind und mit Dampf beheizte Wärmeaustauscher enthalten, wobei der den ersten Verdampfer (102) heizende Dampf vom Heizkessel (96) kommt und der die folgenden Verdampfer heizende Dampf von den vorhergehenden Verdampfern kommt und das gelöste Lignin in der entgegengesetzten Richtung befördert wird.

7. Verfahren nach Anspruch 6, wobei das gelöste Lignin, das die Gruppe der Verdampfer (98, 100, 102) verläßt, weiterhin, bevor es verbrannt wird, im Rauchgas des Heizkessels (96) getrocknet wird.

8. Verfahren nach einem der Ansprüche 1 bis 7, wobei der den Heizkessel (96) verlassende Dampf in eine Gegendruckturbine (130) zum Erzeugen von elektrischem Strom und zum zur Verfügung Stellen von Prozeßdampf eingespeist wird.

**Revendications**

1. Procédé de fabrication d'alcool en circuit fermé à partir d'un matériau lignocellulosique broyé tel que bois, paille, bagasse, balle de céréales, coquille de noix et autres, procédé dans lequel l'apport d'une source d'énergie supplémentaire pour la production de chaleur n'est pas nécessaire ou seulement en quantité très faible, ce procédé devant se dérouler selon les étapes de fabrication suivantes:

a) production d'une réaction entre la matériau lignocellulosique broyé et un mélange d'anhydride sulfureux et d'eau sous une pression et une température obtenues par introduction de vapeur (en 10, 12);

b) mélange du matériau soumis à la réaction de l'étape a) avec une solution alcaline pour dissoudre la lignine soumise à cette réaction (en 22);

c) lavage du matériau de l'étape b) pour séparer la lignine dissoute de la masse cellulosique du mélange (en 28);

d) évaporation d'une quantité suffisante d'eau de la solution de lignine pour obtenir un produite apte à la combustion (en 98, 100, 102), et brûlage de la lignine combustible dans une chaudière (96) pour l'obtention de la vapeur qui sera recyclée à l'étape a) (par 134), et pour l'obtention de carbonate de sodium qui, après dissolution dans l'eau, sera recyclé à l'étape b);

e) hydrolyse de la masse cellulosique de l'étape c) (en 54, 56, 58); et

f) fermentation de la cellulose hydrolysée de l'étape e) pour l'obtention d'alcool (en 70, 74).

2. Procédé selon la revendication 1, dans lequel le mélange de la réaction en a) est constitué d'environ 60% en matière sèche du matériau lignocellulosique broyé, 40% d'eau et 2,16 à 4,32% d'anhydride sulfureux.

3. Procédé selon la revendication 2, dans lequel la réaction de l'étape a) est effectuée à une température située entre 49 et 82°C et une pression entre 9500 et 10500 hPa pour une durée d'environ 40 minutes à une heure.

4. Procédé selon une des revendications 1 à 3, dans lequel l'étape c) est réalisée à l'aide d'un filtre de lavage sous vide (28).

5. Procédé selon une des revendications 1 à 4, dans lequel l'étape a) est réalisée dans un digester rotatif (10, 12).

6. Procédé selon une des revendications 1 à 5, dans lequel l'étape d) est réalisée dans un groupe d'évaporateurs (98, 100, 102) disposés en série et contenant des échangeurs de chaleur chauffés à la vapeur qui arrive, pour le premier évaporateur (102), directement de la chaudière (96), et, pour les évaporateurs suivants, des évaporateurs précédents, la lignine dissoute étant transportée à contre-courant.

7. Procédé selon la revendication 6, dans lequel la lignine dissoute qui quitte le groupe d'évaporateurs (98, 100, 102), est en outre, avant d'être brûlée, séchée, dans les gaz de combustion de la chaudière (96).

8. Procédé selon une des revendications 1 à 7, dans lequel la vapeur quittant la chaudière (96) alimente une turbine à réaction (130) pour la production de courant électrique et l'approvisionnement en vapeur de l'installation.